# EUROPEAN PATENT SPECIFICATION

(11) **EP 2 379 165 B1**
(45) Date of publication and mention of the grant of the patent: **14.09.2016**
(21) Application number: 09752640.4
(22) Date of filing: 09.11.2009
(51) Int. Cl.: A61N 1/05

(54) **IMPLANTABLE AND REFILLABLE DRUG DELIVERY RESERVOIR**
IMPLANTIERBARES UND AUFFÜLLBARES ARZNEIABGABE-RESERVOIR
RÉSERVOIR IMPLANTABLE ET RECHARGEABLE POUR LA DÉLIVRANCE D'UN MÉDICAMENT

(30) Priority: 10.11.2008 US 112818 P
(43) Date of publication of application: 26.10.2011
(73) Proprietor: MED-EL Elektromedizinische Geräte GmbH, 6020 Innsbruck (AT)
(72) Inventor: JOLLY, Claude, A-6020 Innsbruck (AT); HESSLER, Roland, A-6020 Innsbruck (AT)
(74) Representative: Lucke, Andreas
(86) International application number: PCT/US2009/063677
(87) International publication number: WO 2010/054281

(56) References cited:
- WO-A-02/102278
- US-A1- 2003 097 121
- US-A1- 2007 255 237

## Description

This application claims priority from U.S. Provisional Patent 61/112,818, filed November 10, 2008, which is incorporated herein by reference.

### FIELD OF THE INVENTION

The present invention relates to medical implants, and more specifically to implantable drug delivery systems.

### BACKGROUND ART

There is increasing interest in implantable drug delivery systems to deliver therapeutic drugs to targeted internal tissues. Drug eluting electrode leads with cortico steroids have been used successfully in the past with cardiac pacemaker electrodes to reduce the contact impedance. In addition, silicone elastomer loaded with a pharmacological agent has been used as an eluting structure in several applications such as birth control, vascular injury treatment, and stents. There also have been attempts to deliver medicine to the inner ear, for example to promote healing after implantation of cochlear implant electrode.

U.S. Patent 7,044,942 (incorporated herein by reference) describes an implantable system for delivering therapeutic drugs which includes an implantable stimulation electrode which can deliver one or more therapeutic drugs to the surrounding tissue. There also is a separate implantable reservoir for containing the therapeutic drugs and supplying them to the electrode. The patent discusses that the reservoir may be refillable.
US 2003/0097121 A1 discloses a drug delivery catheter having single outlets for fluid delivery, which each may include two outlet channels arranged 180° apart. The outlet channels are covered by a drug-permeable surface for diffusion of a therapeutic drug into a nearby tissue.
WO 02/102278 A2 discloses a drug delivery device having a delivery lumen with a proximal section and a distal section. The proximal end of the device comprises an injection port including a septum. At the distal end of the distal section, a delivery tip is formed which comprises pores or fenestrations through which liquid formulation may flow out of the tip portion and into a round window niche. Further, an overflow lumen extends from the delivery tip at the distal end back to an outlet which is just at the boundary of the proximal and distal sections. In the event that an amount of liquid injected at the injection port exceeds the total capacity of the device, excess liquid flows from the delivery tip into the overflow lumen and out of the outlet.

### SUMMARY OF THE INVENTION

The present invention provides an implantable drug delivery apparatus according to claim 1. Preferable embodiments are defined in the dependent claims.

In one arrangement described herein, a subcutaneous primary reservoir holds a primary volume of a therapeutic drug and is in fluid communication with an input port septum for receiving the therapeutic drug. A subcutaneous secondary reservoir is in fluid communication with the primary reservoir and holds a secondary volume of the therapeutic drug. The secondary reservoir includes a drug permeable surface for diffusion of the therapeutic drug into nearby tissue, and a priming septum for fluid exchange within the secondary reservoir.

Further examples described herein may also include a diffusion channel providing the fluid communication between the primary reservoir and the secondary reservoir. There also may be a channel coil surrounding the diffusion channel for inducing ionic displacement of fluid within the diffusion channel from one reservoir to the other.

The secondary reservoir may form a delivery catheter enclosing the secondary volume. The delivery catheter may contain a semi-permeable filter for mechanically filtering fluid flow through the catheter, for example, using a filter rod based on a drug eluting polymer or gel. The delivery catheter may include a silicone matrix superstructure.

The apparatus may also include an input channel providing fluid communication between the input port septum and the primary reservoir. There also may be an output port septum in communication with the primary reservoir for removing fluid from the primary reservoir, for example based on an output channel providing fluid communication between the output port septum and the primary reser-voir.

At least one of the reservoirs may include at least one internal control surface promoting controlled flow of fluid within the at least one of the reservoirs. For example, one or more internal control surface surfaces may be arranged in a control labyrinth. Or a capillary tube arrangement may be used for controlling flow.

Further examples described herein may include a pressure control element between the primary reservoir and the secondary reservoir preventing pressure transients between the primary reservoir and the secondary reservoir. For example, the pressure control element may include a check valve arrangement or a capillary tube arrangement.

Embodiments of the present invention also include an implantable drug delivery having an input port septum for receiving a therapeutic drug. An implantable delivery catheter holds a volume of the therapeutic drug and has a proximal end in fluid communication with the input port septum, a distal end including an output septum for removing fluid from the catheter, and a drug permeable surface for diffusion of the therapeutic drug into nearby tissue. Specific embodiments may also include an input channel providing fluid communication between the input port septum and the delivery catheter.

In such an embodiment, the delivery catheter may be connected to an implantable stimulator housing containing signal processing circuitry. For example, the implantable stimulator housing may be part of a cochlear implant system and the delivery catheter may be an element of an implantable stimulation electrode.

The drug permeable surface may include a drug permeable membrane. Or there may be an arrangement of drug permeable slits and/or holes. The therapeutic drug fluid may be mixed with an osmotic agent such as a saline solution for accelerating diffusion of the therapeutic drug out of the secondary reservoir.

A charge driver arrangement may be used to apply electric signals such as charge balanced asymmetric pulses for displacing the therapeutic drug within the apparatus by driving electrically charged molecular substances within the therapeutic drug. For example there may be a pair of charge driver electrodes such as an active electrode in the primary reservoir and a ground electrode in the secondary reservoir displaces the therapeutic drug from the primary reservoir to the secondary reservoir over time to replenish the secondary reservoir. Or there may be one electrode within a reservoir and the other electrode outside to the secondary reservoir to displace the therapeutic drug from the secondary reservoir into the nearby tissue.

There may be a magnetic driver arrangement such as a pair of magnets to apply magnetic forces for displacing the therapeutic drug within the apparatus by driving magnetic molecular substances within the therapeutic drug. For example, the magnetic driver arrangement may include a repeller magnet in the primary reservoir and an attractor magnet in the secondary reservoir that displace the therapeutic drug from the primary reservoir to the secondary reservoir over time to replenish the secondary reservoir. Or the drive arrangement may include a magnet within one of the reservoirs and a magnet outside the secondary reservoir which may exert a magnetic attractive force on magnetic molecular substances within the therapeutic drug pulling them through the drug permeable surface into the nearby tissue.

The delivery catheter may contain a semi-permeable filter for mechanically filtering fluid flow through the catheter. For example, the semi-permeable filter may be a structural rod based on a drug eluting polymer or gel. The catheter may also include a silicone matrix superstructure. The drug permeable surface may include a drug permeable membrane and/or drug permeable slits or holes.

Also disclosed herein is an apparatus for transferring fluid containing a therapeutic drug. A delivery syringe has a delivery piston for injecting a delivery volume of therapeutic drug fluid into an implanted system. A receiver syringe has a receiver piston for removing a withdrawal volume of fluid from the implanted system. A piston coupling rod rotates about a center coupling axis and is connected to each of the pistons so that when the delivery piston is pushed into the delivery syringe, the coupling rod rotates to push out the receiver piston the same amount. In some embodiments, a syringe housing may contain both the delivery syringe and the receiver syringe.

Further disclosed herein is a method of delivering a therapeutic drug to an implanted system. A needle of a delivery syringe containing the therapeutic drug is inserted into an input port septum of an implantable primary reservoir. Another needle of a receiver syringe is inserted into a priming septum of an implantable secondary reservoir in fluid communication with the primary reservoir. The therapeutic drug is injected from the delivery syringe into the primary reservoir, and fluid is withdrawn from the secondary reservoir into the receiver syringe so as to prime the reservoirs with the therapeutic drug.

In further specific methods, the needles may be removed from the septums after the reservoirs have been primed, and the reservoirs can be subcutaneously implanted in a selected position in a patient after the needles have been removed. For example, they may be implanted adjacent to the skull of the patient.

Examples of another method of introducing a therapeutic drug in an implanted drug reservoir arrangement start by inserting a needle of a delivery syringe containing the therapeutic drug through the skin of a patient into an input port septum of an implanted drug reservoir. Another needle of a receiver syringe is inserted through the skin of the patient into an output septum of the implanted drug reservoir. The therapeutic drug is injected from the delivery syringe into the drug reservoir, and fluid is withdrawn from the drug reservoir into the receiver syringe so as to introduce the therapeutic drug into the drug reservoir. The needles may be removed from the septums after the therapeutic drug has been introduced into the drug reservoir.

### BRIEF DESCRIPTION OF THE DRAWINGS

Figure 1 shows an embodiment of an implantable drug delivery system.
Figure 2 shows another embodiment of an implantable drug delivery system using a charge drive arrangement.
Figure 3 shows principles of a magnetic drive arrangement as used by another embodiment of the present invention.
Figure 4 shows an embodiment having a volume of therapeutic drug fluid in a delivery catheter.
Figure 5 shows an embodiment having a semi-permeable filter in the form of a drug delivery rod.
Figure 6 shows an embodiment in which an implantable delivery catheter is connected to the body of an implant housing.
Figure 7 shows another embodiment having a primary reservoir and a secondary reservoir connected by a diffusion channel.
Figure 8 shows an embodiment having one or more internal control surfaces to form a control labyrinth that promotes controlled flow of fluid.
Figure 9 shows an embodiment having a pressure control element between the primary reservoir and the secondary reservoir for preventing pressure transients.
Figure 10 shows a device having a delivery syringe and a receiver syringe in accordance with an embodiment of the present invention.

### DETAILED DESCRIPTION OF SPECIFIC EMBODIMENTS

Various embodiments of the present invention are directed to implantable drug delivery apparatuses and methods. Embodiments include a fillable and refillable implantable drug delivery system which does not increase its internal pressure while refilling. And electrical and/or magnetic pulses can be used to displace molecules within the therapeutic drug within the apparatus. Such embodiments and techniques are useful for delivering a solution of a therapeutic drug into target tissue such as a body cavity like the cochlea. Embodiments also include one or more subcutaneous drug delivery reservoirs that are transcutaneously refillable without increasing pressure. Embodiments also maintain homogeneity of a therapeutic drug within a drug delivery reservoir as the drug diffuses to the outside of the reservoir.

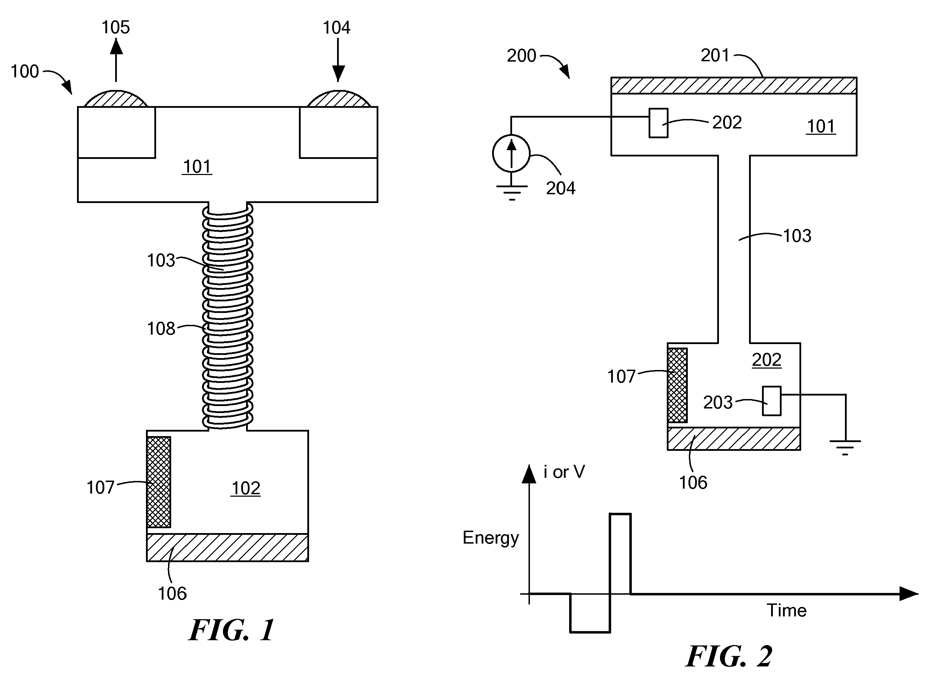

Figure 1 shows an embodiment of an implantable drug delivery system **100** that includes a subcutaneous primary reservoir **101** that holds a primary volume of a therapeutic drug. The primary reservoir **101** includes an input port septum **104** for receiving the therapeutic drug and a corresponding an output port septum **105** for removing fluid. A diffusion channel **103** provides a fluid communication path from the primary reservoir **101** to a secondary reservoir **102** that holds a secondary volume of the therapeutic drug. The secondary reservoir **102** includes a drug permeable surface **107** such as a drug permeable membrane, slits or holes for diffusion of the therapeutic drug into nearby tissue, and a priming septum **106** for fluid exchange within the secondary reservoir **102.** It may be useful in such an arrangement to mix the therapeutic drug with an osmotic agent such as a saline solution for promoting and accelerating diffusion of the therapeutic drug out of the secondary reservoir **102.** The embodiment shown in Fig. 1 also includes a channel coil **108** that surrounds the diffusion channel **103** for inducing ionic displacement of fluid within the diffusion channel from one reservoir to the other.

The system may be filled with therapeutic drug fluid before implantation by inserting a needle of a delivery syringe containing the therapeutic drug fluid into the input port septum **104.** A needle of a receiver syringe is inserted into the priming septum **106** and the therapeutic drug fluid is injected from the delivery syringe into the primary reservoir **101** while the receiver syringe withdraws fluid from the secondary reservoir **102,** thereby priming the reservoirs with the therapeutic drug fluid. After priming the system, the needles are removed from the septums and the drug delivery system is ready to be subcutaneously implanted in a selected position in a patient, for example, adjacent to the skull of the patient for use with a cochlear implant system.

The implantable drug delivery system **100** allows refilling of the reservoirs **101** and **102,** either with the same therapeutic drug or with a new therapeutic drug with a different molecular content. The refilling process does not raise pressure either within the internal volume of the drug delivery system **100** or in the surrounding tissue and fluid region outside the secondary reservoir **102.** No special bacterial filter is needed because molecular diffusion preferentially occurs through the ion permeable membrane drug delivery surface **107,** or through punctures in the drug delivery surface **107** that are smaller than bacteria size.

Figure 2 shows another similar embodiment of an implantable drug delivery system **200** which has just a single primary septum **201** on top of the primary reservoir **101.** And instead of a channel coil arrangement, this embodiment uses a charge driver arrangement to for displacing the therapeutic drug within the apparatus by driving electrically charged molecular substances within the therapeutic drug. Specifically, a pulse generator **204** (e.g., from an implantable stimulator) generates a drive signal to an active electrode **202** in the primary reservoir **101.** A ground electrode **203** in the secondary reservoir **102** completes the current path. A drive signal based on charge balanced asymmetric pulses such as the example shown in the bottom of Fig. 2 then can drive electrically charged molecular substances within the therapeutic drug, for example, to displace the therapeutic drug from the primary reservoir **101** to the secondary reservoir **102** over time to replenish the secondary reservoir.

Instead of placing the ground electrode **203** inside the secondary reservoir **102,** in some embodiments it may be external to the secondary reservoir **102.** Such an arrangement allows a drive signal based on charge balanced asymmetric pulses to displace the therapeutic drug from the primary reservoir **101,** through the secondary reservoir **102,** and by active diffusion through the drug permeable surface **107** into the nearby tissue, e.g., into the surrounding cochlear fluid or extra-cellular fluid. Such a charge driver arrangement may be especially effective if there are small ionic channels between the polymer matrix of the drug permeable surface **107** and the surrounding tissue. These can be created by punctures made with a small needle, laser ablation of holes, use of an ion permeable membrane, and/or one or more slits from scalpel, any of which may provide an improved passage for the flow of complex charged molecules in the therapeutic drug to flow from inside the secondary reservoir **102** out into the surrounding fluids and tissues.

Using a balanced charge drive signal may help avoid undesirable corrosion of the electrodes **202** and **203.** The pulse generator **204** advantageously may be located within the housing of a cochlear implant stimulator, which typically are designed to deliver charge balanced symmetric or asymmetric pulses. Alternatively the drive signal may be based on use of tri-phasic pulses to provoke a net charge displacement in one direction of the electrodes. The associated insulated wiring for such embodiments both to and from the pulse generator **204** and between the electrodes **202** and **203** may run within the interior volume of the reservoirs, or within or along the walls and surfaces of the apparatus structures.

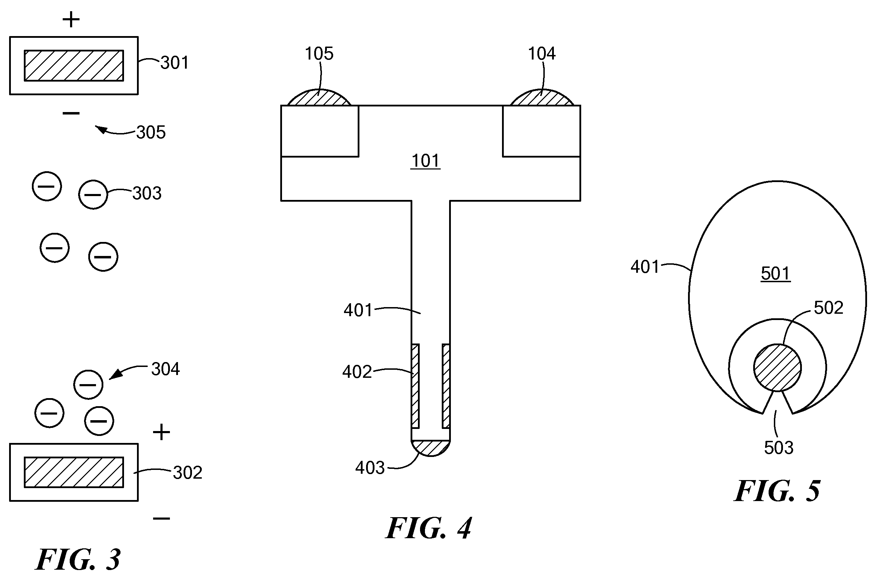

Rather than a charge drive arrangement as depicted in Fig. 2, some embodiments may use a magnetic drive arrangement based on the principles shown in Figure 3 for displacing the therapeutic drug within the apparatus by driving magnetic molecular substances within the therapeutic drug. Instead of electrodes **203** and **204,** a magnetic driver arrangement uses a repeller magnet **301** and an attractor magnet **302** that set up a magnetic field between them that displaces the therapeutic drug within the apparatus by driving magnetic molecular substances **303** within the therapeutic drug. In the example shown in Fig. 3, this drives the magnetic molecules away from the repeller magnet **301** to create a region of lower molecular concentration **305** in the vicinity, while a higher molecular concentration **304** region develops near to the attractor magnet **302** located, for example, outside the secondary reservoir to exert a magnetic attractive force on magnetic molecular substances within the therapeutic drug and pull them through the drug permeable surface into the nearby tissue. Alternatively, the attractor magnet **302** may be located within the secondary reservoir to displace the therapeutic drug fluid from the primary reservoir to the secondary reservoir over time to replenish the secondary reservoir. The magnets **301** and/or **302** may usefully be covered by a protective encapsulation layer.

Figure 4 shows another embodiment of an implantable drug delivery apparatus in which the arrangement of the primary reservoir **101** and its input septum **104** and output septum **105** are as in Fig. 1, but the secondary reservoir **401** is elongated to form a delivery catheter that encloses the secondary volume. Thus, the proximal end of delivery catheter secondary reservoir **401** is in fluid communication with the input septum **104** while the distal end includes an output septum **403** for removing fluid from the secondary reservoir **401** when filling or refilling the system. A drug permeable surface **402** such as an ion permeable diffusion membrane diffuses the therapeutic drug fluid into the surrounding tissues and fluids.

In some embodiments, the interior of the secondary reservoir **401** may include a semi-permeable filter such as the drug delivery rod **502** shown in Figure 5 for mechanically filtering fluid flow through the catheter. In the example shown, the drug delivery rod **502** may be in the specific form of a drug eluting gel or drug eluting polymer such as a drug eluting silicone rod. Such a drug delivery rod **502** is then embedded or incorporated into the main superstructure of the secondary reservoir **401,** which may be, for example, a silicone matrix **501.** The silicone matrix **501** may include a slit opening **503** that allows hydration of the drug eluting material by the surrounding fluid. In some embodiments, the elongated form of the secondary reservoir **401** may be pre-shaped to have a bend in it to accommodate placement around internal body structures. Or the secondary reservoir **401** may be flexible to be bendable around such internal body structures.

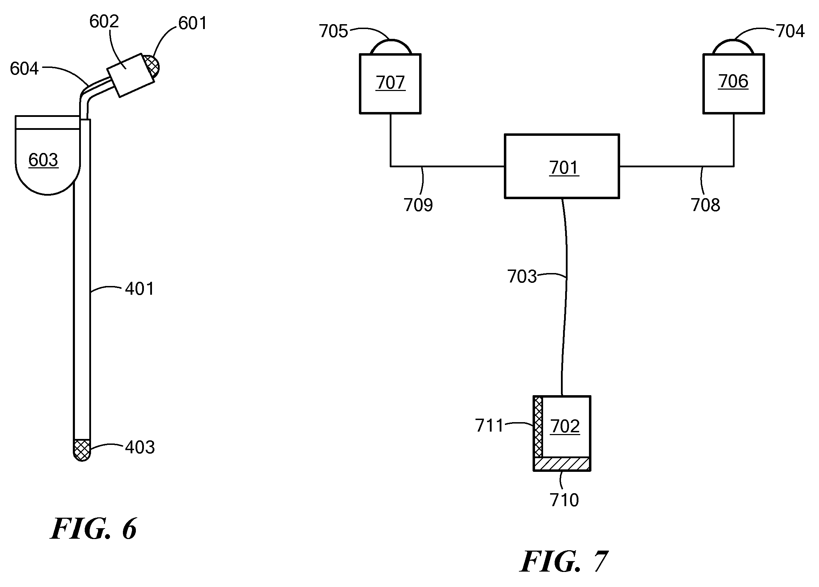

Figure 6 shows another embodiment in which an implantable delivery catheter 401 is connected to the body of an implant housing **603,** such as a cochlear implant stimulator housing. In such an embodiment, the delivery catheter **401** may be an internal volume within a stimulator electrode array which is implanted, for example, in the cochlea of a hearing impaired patient. The electrode array in such an embodiment includes a drug permeable surface as described above (e.g., an ion or molecule permeable membrane and/or an arrangement of multiple diffusion slits or holes) through which the therapeutic drug diffuses into the surrounding tissue or fluid. In the embodiment shown, the therapeutic drug is delivered to the delivery catheter **401** via an arrangement of an input septum **601** over an input port **602,** through an input channel **604** and the implant housing **603.** In another embodiment, there may be simply an input septum **601** without a discernable defined input port **602** as such. In either configuration, priming septum **106** may be used before implantation to withdraw existing fluid within the delivery catheter **401** to initially fill the system. After implantation, additional therapeutic drug fluid can be periodically added transcutaneously through the input septum **601,** which located is just under the skin of the patient.

Figure 7 shows another embodiment having a primary reservoir 701 and a secondary reservoir **702** connected by a diffusion channel **703.** The secondary reservoir **702** includes a priming septum **710** and a drug delivery surface **711** as described above with regards to other embodiments. In addition, an input channel **708** provides fluid communication between the primary reservoir **701** and an input port **706** having an input septum **704.** Similarly, an output channel **709** also provides fluid communication between the primary reservoir **701** and an output port **707** having an output septum **705.**

Initial filling of the system may occur before implantation using the input septum **704** and priming septum **710.** After implantation, the system can be filled/replenished using a delivery syringe containing the therapeutic drug fluid to refill the primary reservoir **701** transcutaneously through the skin of a patient into the input septum **704,** while a receiver syringe under negative pressure (i.e., withdrawing the plunger) permits air or old fluid to be withdrawn transcutaneously through the output septum **705** and the skin into the receiver syringe, thereby refilling/replenishing the primary reservoir **701.** After the therapeutic drug fluid has been introduced into the primary reservoir **701,** both the delivery syringe needle and the receiver needle are removed.

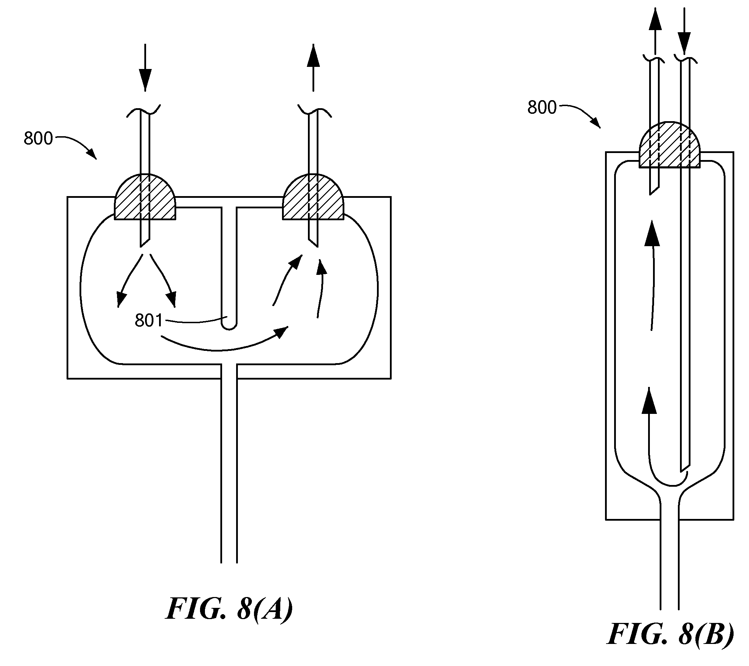

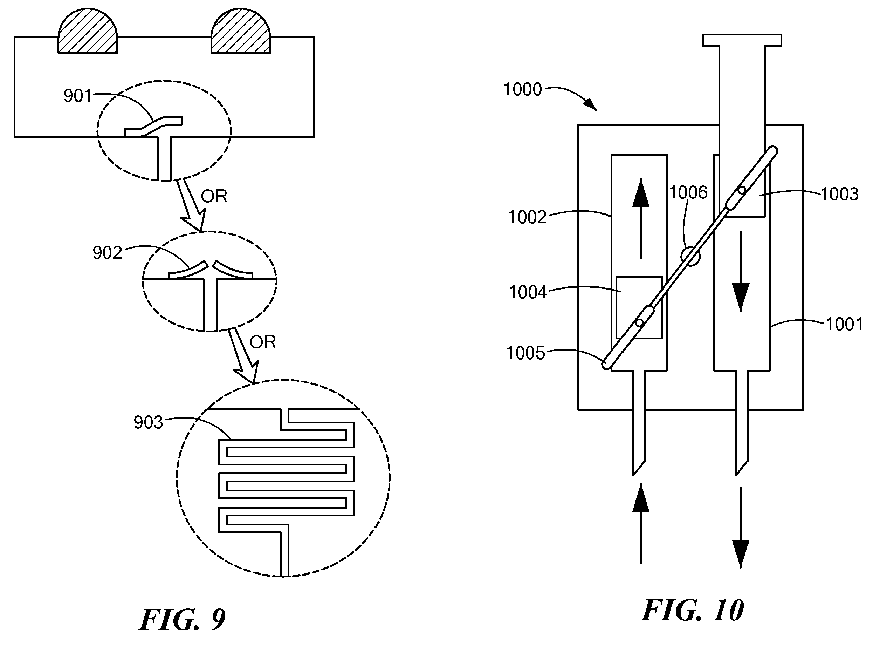

Embodiments may also include an internal flow control arrangement for correctly and reliably directing fluid flow within the primary reservoir, for example to maintain a desired concentration of therapeutic drug within the fluid in the reservoir. For example, Figure 8 shows a primary reservoir **800** having one or more internal control surfaces **801** to form a control labyrinth that promotes controlled flow of fluid within the primary reservoir **800.** Fig. 8A shows use of a single control surface **801,** in other embodiments, there may be multiple such control surfaces arranged to form a more complicated control labyrinth. For example, a capillary tube arrangement such as the one shown at the bottom of Fig. 9 may be used as a control labyrinth within the primary reservoir **800.**

Embodiments may also include a pressure control element between the primary reservoir and the secondary reservoir for preventing pressure transients between the primary reservoir and the secondary reservoir. Limiting pressure transients between the primary reservoir and the secondary reservoir during fluid filling operations also serves to prevent pressure transients in the surrounding tissue and fluid region outside the secondary reservoir. For example, such a pressure control element may be in the specific form of a check valve arrangement at the opening to the diffusion channel such as single flap check valve **901** or double flap check valve **902.** If pressure increases in the primary reservoir during fluid filling, the check valve **901** or **902** closes to prevent a pressure transient in the secondary reservoir. Alternatively, a capillary tube arrangement **903** at the beginning of the diffusion channel may serve the same purpose by allowing diffusion but providing significant resistance to any pressure driven fluid flow.

To maintain constant pressure in the reservoirs, the fluid flow into and out of the system needs to be coordinated in volume and flow rate. This can be achieved with a device or arrangement which takes in and out of the reservoir, the same amount of fluid, at the same time and the same flow rate. Figure 10 shows one example of such a fluid replacement device **1000** which contains in a single housing both a delivery syringe **1001** having a delivery piston **1003** and a receiver syringe **1002** having a receiver piston **1004.** Connected to each piston is a piston coupling rod **1005** which rotates about a coupling axis **1006** so that when the delivery piston is pushed into the delivery syringe **1001,** the coupling rod **1005** rotates to push out the receiver piston **1004** the same amount.

Although various exemplary embodiments of the invention have been disclosed, it should be apparent to those skilled in the art that various changes and modifications can be made which will achieve some of the advantages of the invention.

## Claims

1. An implantable drug delivery apparatus (100) comprising:
an input port septum (104) for receiving a therapeutic drug; and
an implantable delivery catheter (401) holding a volume of the therapeutic drug and having:
a proximal end in fluid communication with the input port septum (104), **characterised in that** the implantable delivery catheter futher comprises
a distal end including an output septum (403) for removing fluid from the catheter when filling or refilling the system, and a drug permeable membrane (402) for diffusion of the therapeutic drug into nearby tissue,
wherein the implantable drug delivery apparatus is configured for being transcutaneously refillable without increasing a pressure within the internal volume and in the surrounding of the drug delivery apparatus.

2. An apparatus according to claim 1, wherein the delivery catheter is connected to an implantable stimulator housing containing signal processing circuitry (204), and/or wherein the implantable stimulator housing is part of a cochlear implant system.

3. An apparatus according to claim 1, wherein the delivery catheter is an element of an implantable stimulation electrode.

4. An apparatus according to claim 3, wherein the stimulation electrode is an element of a cochlear implant system.

5. An apparatus according to claim 1, wherein the delivery catheter contains a semi-permeable filter (502) for mechanically filtering fluid flow through the catheter, wherein the semi-permeable filter preferably includes a drug eluting polymer.

6. An apparatus according to claim 5, wherein the semi-permeable filter (502) includes a drug eluting gel, and/or
wherein the catheter includes a silicone matrix superstructure.

7. An apparatus according to claim 1, further comprising:
an input channel providing fluid communication between the input port septum and the delivery catheter.

8. An apparatus according to claim 1, wherein the therapeutic drug is mixed with an osmotic agent for accelerating diffusion of the therapeutic drug out of the delivery catheter.

9. An apparatus according to claim 1, further comprising:
a charge driver arrangement applying electric signals for displacing the therapeutic drug within the apparatus by driving electrically charged molecular substances within the therapeutic drug.

10. An apparatus according to claim 9, wherein the charge driver arrangement includes a pair of charge driver electrodes (202, 203),
wherein the charge driver electrodes preferably include an active electrode (202) inside the catheter and a ground electrode (203) outside the catheter.

11. An apparatus according to claim 9, wherein the electric signals include charge balanced asymmetric pulses.

12. An apparatus according to claim 9, wherein the charge driver arrangement displaces the therapeutic drug from the catheter into the nearby tissue.

13. An apparatus according to claim 1, further comprising:
a magnetic driver arrangement applying magnetic forces for displacing the therapeutic drug within the apparatus by driving magnetic molecular substances within the therapeutic drug,
wherein the magnetic driver arrangement preferably displaces the therapeutic drug from the catheter into the nearby tissue.

14. An apparatus according to claim 13, wherein the magnetic driver arrangement includes at least one attractor magnet (302) outside the catheter exerting a magnetic attractive force on magnetic molecular substances within the therapeutic drug pulling them through the drug permeable surface into the nearby tissue.

15. An apparatus according to claim 13, wherein the magnetic driver arrangement includes a pair of driver magnets for displacing the therapeutic drug within the apparatus,
wherein the magnets preferably include a repeller magnet (301) within the catheter and an attractor magnet (302) outside the catheter that displace the therapeutic drug from the catheter to the nearby tissue.

## Patentansprüche

1. Implantierbare Medikamentenabgabevorrichtung (100), die Folgendes umfasst:
ein Eingangsport-Septum (104) zum Empfangen eines therapeutischen Medikaments; und
einen implantierbaren Abgabekatheter (401) der ein Volumen des therapeutischen Medikaments aufnimmt und Folgendes umfasst:
ein proximales Endes, welches sich in Fluidkommunikation mit dem Eingangsport-Septum (104) befindet,
**dadurch gekennzeichnet, dass** der implantierbare Abgabekatheter ferner ein distales Ende umfasst, welches ein Ausgangs-Septum (403) umfasst, zum Entfernen von Fluid aus dem Katheter, wenn das System befüllt oder wiederbefüllt wird, und
eine medikamentenpermeable Membran (402) zur Diffusion des therapeutischen Medikaments in das naheliegende Gewebe,
wobei die implantierbare Medikamenten-Abgabevorrichtung dazu konfiguriert ist, transkutan wiederbefüllbar zu sein, ohne einen Druck innerhalb des inneren Volumens und in der Umgebung der Medikamentenabgabevorrichtung zu erhöhen.

2. Vorrichtung nach Anspruch 1, bei der der Abgabekatheter mit einem implantierbaren Stimulatorgehäuse verbunden ist, welches eine Signalverarbeitungsschaltung (204) umfasst, und/oder bei der das implantierbare Stimulatorgehäuse Teil eines Cochlearimplantatsystems ist.

3. Vorrichtung nach Anspruch 1, bei der der Abgabekatheter ein Element einer implantierbaren Stimulationselektrode ist.

4. Vorrichtung nach Anspruch 3, bei der die Stimulationselektrode ein Element eines Cochlearimplantatsystems ist.

5. Vorrichtung nach Anspruch 1, bei der der Abgabekatheter einen semi-permeablen Filter (502) zum mechanischen Filtern des Fluidflusses durch den Katheter umfasst, wobei der semi-permeable Filter vorzugsweise ein Medikamenten-eluierendes Polymer umfasst.

6. Vorrichtung nach Anspruch 5, bei der der semi-permeable Filter (502) ein Medikamenten-eluierendes Gel umfasst, und/oder bei der der Katheter eine Silikonmatrixsuperstruktur umfasst.

7. Vorrichtung nach Anspruch 1, die ferner Folgendes umfasst:
einen Eingangskanal, der eine Fluidkommunikation zwischen dem Eingangsport-Septum und dem Abgabekatheter bereitstellt.

8. Vorrichtung nach Anspruch 1, bei der das therapeutische Medikament mit einem osmotischen Mittel gemischt ist, um die Diffusion des therapeutischen Medikaments aus dem Abgabekatheter zu beschleunigen.

9. Vorrichtung nach Anspruch 1, die ferner Folgendes umfasst:
eine Ladungstreiberanordnung, die elektrische Signale angelegt zum Verlagern des therapeutischen Medikaments innerhalb der Vorrichtung, indem elektrisch geladene molekulare Substanzen innerhalb des therapeutischen Medikaments angetrieben werden.

10. Vorrichtung nach Anspruch 9, bei der die Ladungstreiberanordnung ein Paar von Ladungstreiberelektroden (202, 203) umfasst,
wobei die Ladungstreiberelektroden vorzugsweise eine aktive Elektrode (202) innerhalb des Katheters und eine Erdungs-Elektrode (203) außerhalb des Katheters umfassen.

11. Vorrichtung nach Anspruch 9, bei der die elektrischen Signale ladungsbalancierte asymmetrische Pulse umfassen.

12. Vorrichtung nach Anspruch 9, bei der die Ladungstreiberanordnung das therapeutische Medikament aus dem Katheter in das naheliegende Gewebe verlagert.

13. Vorrichtung nach Anspruch 1, die ferner Folgendes umfasst:
eine magnetische Treiberanordnung, die magnetische Kräfte anlegt zum Verlagern des therapeutischen Medikaments innerhalb der Vorrichtung, indem magnetische molekulare Substanzen innerhalb des therapeutischen Medikaments angetrieben werden,
wobei die magnetische Treiberanordnung vorzugsweise das therapeutische Medikament von dem Katheter in das nahegelegene Gewebe verlagert.

14. Vorrichtung nach Anspruch 13, bei der die magnetische Treiberanordnung mindestens einen Attraktor-Magneten (302) außerhalb des Katheters umfasst, der eine magnetische Anziehungskraft auf die magnetischen molekularen Substanzen innerhalb des therapeutischen Medikaments ausübt, die diese durch die medikamenten-permeable Oberfläche in das nahegelegene Gewebe zieht.

15. Vorrichtung nach Anspruch 13, bei der die magnetische Treiberanordnung ein Paar von Treibermagneten zum Verlagern des therapeutischen Medikaments innerhalb der Vorrichtung umfasst,
wobei die Magneten vorzugsweise einen Abstoßmagneten (301) innerhalb des Katheters und einen Attraktor-Magneten (302) außerhalb des Katheters umfassen, die das therapeutische Medikament von dem Katheter in das nahegelegene Gewebe verlagern.

## Revendications

1. Appareil de délivrance de médicament implantable (100) comprenant :
un septum d'orifice d'entrée (104) pour recevoir un médicament thérapeutique ; et
un cathéter de délivrance implantable (401) contenant un volume du médicament thérapeutique et ayant :
une extrémité proximale en communication fluide avec le septum d'orifice d'entrée (104),
**caractérisé en ce que** le cathéter de délivrance implantable comprend
une extrémité distale incluant un septum de sortie (403) pour retirer le fluide du cathéter lors du remplissage ou re-remplissage du système, et
une membrane perméable au médicament (402) pour diffusion du médicament thérapeutique dans le tissu voisin,
dans lequel l'appareil de délivrance de médicament immplantable est configuré pour être remplissable par voie transcutanée sans augmenter une pression à l'intérieur du volume interne et dans le voisinage de l'appareil de délivrance de médicament.

2. Appareil selon la revendication 1, dans lequel le cathéter de délivrance est relié à un logement stimulateur implantable contenant un ensemble de circuits de traitement de signaux (204), et/ou
dans lequel le logement stimulateur implantable fait partie d'un système d'implant cochléaire.

3. Appareil selon la revendication 1, dans lequel le cathéter de délivrance est un élément d'une électrode de stimulation implantable.

4. Appareil selon la revendication 3, dans lequel l'électrode de stimulation est un élément d'un système d'implant cochléaire.

5. Appareil selon la revendication 1, dans lequel le cathéter de délivrance contient un filtre semi-perméable (502) pour filtrer mécaniquement l'écoulement de fluide à travers le cathéter,
dans lequel le filtre semi-perméable comprend de préférence un polymère d'élution de médicament.

6. Appareil selon la revendication 5, dans lequel le filtre semi-perméable (502) comprend un gel d'élution de médicament, et/ou
dans lequel le cathéter comprend une superstructure à matrice de silicium.

7. Appareil selon la revendication 1, comprenant en outre :
un canal d'entrée assurant une communication fluide entre le septum d'orifice d'entrée et le cathéter de délivrance.

8. Appareil selon la revendication 1, dans lequel le médicament thérapeutique est mélangé avec un agent osmotique pour accélérer la diffusion du médicament thérapeutique hors du cathéter de délivrance.

9. Appareil selon la revendication 1, comprenant en outre :
un agencement d'entraînement de charge appliquant des signaux électriques pour déplacer le médicament thérapeutique à l'intérieur de l'appareil en entraînant des substances moléculaires chargées électriquement à l'intérieur du médicament thérapeutique.

10. Appareil selon la revendication 9, dans lequel l'agencement d'entraînement de charge comprend une paire d'électrodes d'entraînement de charge (202, 203),
dans lequel les électrodes d'entraînement de charge comprennent de préférence une électrode active (202) à l'intérieur du cathéter et une électrode de masse (203) à l'extérieur du cathéter.

11. Appareil selon la revendication 9, dans lequel les signaux électriques comprennent des impulsions asymétriques équilibrées en charge.

12. Appareil selon la revendication 9, dans lequel l'agencement d'entraînement de charge déplace le médicament thérapeutique du cathéter dans le tissu voisin.

13. Appareil selon la revendication 1, comprenant en outre :
un agencement d'entraînement magnétique appliquant des forces magnétiques pour le déplacement du médicament thérapeutique à l'intérieur de l'appareil en entraînant des substances moléculaires magnétiques à l'intérieur du médicament thérapeutique,
dans lequel l'agencement d'entraînement magnétique déplace de préférence le médicament thérapeutique du cathéter dans le tissu voisin.

14. Appareil selon la revendication 13, dans lequel l'agencement d'entraînement magnétique comprend au moins un aimant attracteur (302) à l'extérieur du cathéter exerçant une force attractive magnétique sur les substances moléculaires magnétiques à l'intérieur du médicament thérapeutique, les tirant à travers la surface perméable au médicament dans le tissu voisin.

15. Appareil selon la revendication 13, dans lequel l'agencement d'entraînement magnétique comprend une paire d'aimants d'entraînement pour déplacer le médicament thérapeutique à l'intérieur de l'appareil,
dans lequel les aimants comprennent de préférence un aimant répulseur (301) à l'intérieur du cathéter et un aimant attracteur (302) à l'extérieur du cathéter qui déplacent le médicament thérapeutique du cathéter vers le tissu voisin.
